# EUROPEAN PATENT SPECIFICATION

(11) **EP 2 977 042 B1**
(45) Date of publication and mention of the grant of the patent: **01.01.2020**
(21) Application number: 15185071.6
(22) Date of filing: 07.02.2007
(51) Int. Cl.: A61K 9/00, A61K 31/335, A61K 47/02, A61K 47/18, A61K 9/08

(54) **OLOPATADINE FORMULATIONS FOR TOPICAL NASAL ADMINISTRATION**
FORMULIERUNGEN VON OLOPATADIN ZUR TOPISCHEN NASALEN VERABREICHUNG
FORMULATIONS À BASE D'OLOPATADINE POUR ADMINISTRATION NASALE TOPIQUE

(43) Date of publication of application: 27.01.2016
(62) Divisional of application: 07750153.4
(73) Proprietor: Novartis AG, 4056 Basel (CH)
(72) Inventor: Singh, Onkar, N., Arlington, TX Texas 76017 (US); Wall, G., Michael, Fort Worth, TX Texas 76109 (US); Jani, Rajni, Fort Worth, TX Texas 76109 (US); Chowhan, Masood A., Arlington, TX Texas 76016 (US); Han, Wesley, Wehsin, Arlington, TX Texas 76006 (US)
(74) Representative: Stierwald, Michael

(56) References cited:
- WO-A-01/54687
- WO-A-03/002093
- WO-A-2004/043470

## Description

### BACKGROUND OF THE INVENTION

### Field of the Invention

The present invention relates to topical formulations used for treating allergic and inflammatory diseases. More particularly, the present invention relates to formulations of olopatadine and their use for treating and/or preventing allergic or inflammatory disorders of the nose.

### Description of the Related Art

As taught in U.S. Patent Nos. 4,871,865 and 4,923,892, both assigned to Burroughs Wellcome Co. ("the Burroughs Wellcome Patents"), certain carboxylic acid derivatives of doxepin, including olopatadine (chemical name: Z-11-(3-dimethylaminopropylidene)-6, 11-dihydrodibenz[b,e]oxepine-2-acetic acid), have antihistamine and antiasthmatic activity. These two patents classify the carboxylic acid derivatives of doxepin as mast cell stabilizers with antihistaminic action because they are believed to inhibit the release of autacoids (i.e., histamine, serotonin, and the like) from mast cells and to inhibit directly histamine's effects on target tissues. The Burroughs Wellcome Patents teach various pharmaceutical formulations containing the carboxylic acid derivatives of doxepin, including nasal spray and ophthalmic formulations. See, for example, Col. 7, lines 7 - 26, and Examples 8 (H) and 8 (I) of the '865 patent.

U.S. Patent No. 5,116,863, assigned to Kyowa Hakko Kogyo Co., Ltd., ("the Kyowa patent"), teaches that acetic acid derivatives of doxepin and, in particular, olopatadine, have anti-allergic and anti-inflammatory activity. Olopatadine is the *cis* form of the compound having the formula: 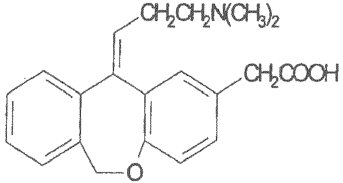 Medicament forms taught by the Kyowa patent for the acetic acid derivatives of doxepin include a wide range of acceptable carriers; however, only oral and injection administration forms are mentioned.

U.S. Patent No. 5,641,805, assigned to Alcon Laboratories, Inc. and Kyowa Hakko Kogyo Co., Ltd., teaches topical ophthalmic formulations containing olopatadine for treating allergic eye diseases. According to the '805 patent, the topical formulations may be solutions, suspensions or gels. The formulations contain olopatadine, an isotonic agent, and "if required, a preservative, a buffering agent, a stabilizer, a viscous vehicle and the like." See Col. 6, lines 30 - 43. "[P]olyvinyl alcohol, polyvinylpyrrolidone, polyacrylic acid or the like" are mentioned as the viscous vehicle. See Col. 6, lines 55 - 57.

WO 03/002093 A1 teaches aqueous formulations of olopatadine stabilised with polyvinylpyrrolidone or polystyrene sulfonic acid.

PATANOL® (olopatadine hydrochloride ophthalmic solution) 0.1% is currently the only commercially available olopatadine product for ophthalmic use. According to its labelling information, it contains olopatadine hydrochloride equivalent to 0.1% olopatadine, 0.01% benzalkonium chloride, and unspecified amounts of sodium chloride, dibasic sodium phosphate, hydrochloric acid and/or sodium hydroxide (to adjust pH) and purified water.

Topical olopatadine formulations that are effective as products for treating allergic or inflammatory conditions in the nose are desirable.

### Summary of the Invention

The present invention provides topical olopatadine formulations that are effective as products for treating allergic or inflammatory disorders of the nose. The formulations of the present invention are aqueous solutions that comprise approximately 0.6 % olopatadine. Despite their relatively high concentration of olopatadine, they do not contain any polymeric ingredient as a physical stability enhancing ingredient. The formulations contain a phosphate salt that permits the pH of the formulations to be maintained within the range 3.5 - 3.95 and that also aids in solubilizing the olopatadine drug in the presence of sodium chloride.

Among other factors, the present invention is based on the finding that stable, nasal spray, solution formulations of olopatadine can be prepared within a pH range of 3.5 - 3.95 using a phosphate buffer without the need for any polymeric ingredient to enhance the solubility or physical stability of the formulation.

### Brief Description Of The Drawings

Figures 1A and 1B show the pH-solubility profile of olopatadine.
Figure 2 shows the effect of NaCl and Na₂HPO₄ on the dissolution of olopatadine in water.
Figure 3 shows the effect of NaCl and Na₂HPO₄ on the dissolution of olopatadine in a nasal vehicle.
Figure 4 shows the effect of NaCl and Na₂HPO₄ concentrations on the dissolution rate of olopatadine in a nasal vehicle.
Figure 5 shows the buffer capacity of an olopatadine nasal spray composition.

### Detailed Description of the Invention

Unless indicated otherwise, all component amounts are presented on a % (w/v) basis and all references to amounts of olopatadine are to olopatadine free base.

Olopatadine is a known compound that can be obtained by the methods disclosed in U.S. Patent No. 5,11 6,863. The solution formulations of the present invention contain 0.54 - 0.62% olopatadine. Preferably, the solution formulations contain 0.6% olopatadine.

Olopatadine has both a carboxylic functional group (pKa₁ = 4.18) and a tertiary amino group (pKa₂ = 9.79). It exists in different ionic forms depending upon the pH of the solution. Olopatadine exists predominantly as a zwitterion in the pH range between the two pKa values with a negatively-charged carboxylic group and a positively-charged tertiary amino group. The isoelectric point of the olopatadine zwitterion is at pH 6.99. At a pH lower than pKa₁, cationic olopatadine (with ionized tertiary amino group) is dominant. At a pH higher than pKa₂, anionic olopatadine (with ionized carboxylic group) is dominant.

### Acid-Base Equilibrium of Olopatadine

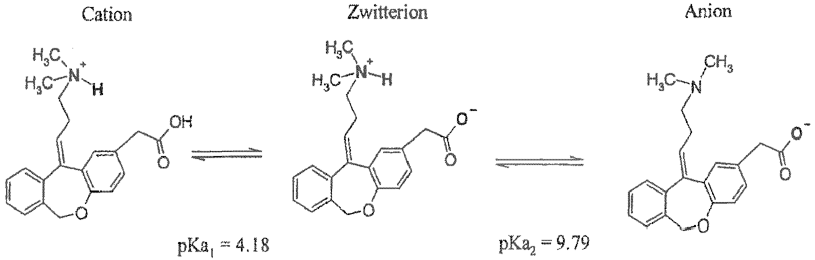

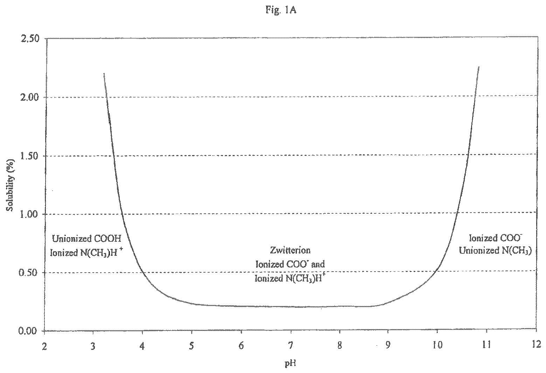

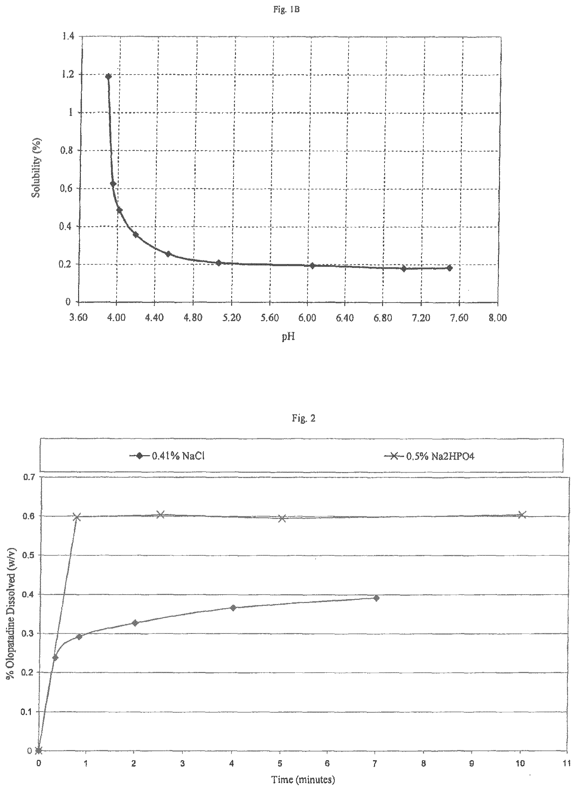

In many zwitterionic molecules, such as various amino acids, intra-molecular ionic interactions are not significant or do not exist. But the structure of olopatadine is such that intra-molecular interactions exist and are significant, possibly due to the distance and bonding angle between the oppositely charged functional groups. This interaction effectively reduces the ionic and dipole character of the molecule. The net effect of the intra-molecular interactions between the oppositely charged functional groups is the reduction of aqueous solubility of olopatadine. Olopatadine has the pH-solubility profile shown in Figures 1A (theoretical) and 1B (obtained using phosphate buffer).

Generally, olopatadine will be added in the form of a pharmaceutically acceptable salt. Examples of the pharmaceutically acceptable salts of olopatadine include inorganic acid salts such as hydrochloride, hydrobromide, sulfate and phosphate; organic acid salts such as acetate, maleate, fumarate, tartrate and citrate; alkali metal salts such as sodium salt and potassium salt; alkaline earth metal salts such as magnesium salt and calcium salt; metal salts such as aluminum salt and zinc salt; and organic amine addition salts such as triethylamine addition salt (also known as tromethamine), morpholine addition salt and piperidine addition salt. The most preferred form of olopatadine for use in the solution compositions of the present invention is the hydrochloride salt of (Z)-11-(3-dimethylaminopropylidene)-6,11-dihydro-dibenz-[b,e]oxepin-2-acetic acid. When olopatadine is added to the compositions of the present invention in this salt form, 0.665% olopatadine hydrochloride is equivalent to 0.6% olopatadine free base. Preferably the compositions of the present invention comprise approximately 0.665% olopatadine hydrochloride.

In addition to olopatadine, the aqueous solution compositions of the present invention comprise a phosphate salt. The phosphate salt not only helps maintain the pH of the compositions within the targeted pH range of 3.5 - 3.95 by contributing to the buffer capacity of the compositions, but also helps solubilize olopatadine. Suitable phosphate salts for use in the compositions of the present invention include monobasic sodium phosphate, dibasic sodium phosphate, tribasic sodium phosphate, monobasic potassium phosphate, dibasic potassium phosphate, and tribasic potassium phosphate. The most preferred phosphate salt is dibasic sodium phosphate. The compositions of the present invention comprise an amount of phosphate salt equivalent (on an osmolality contribution basis) to 0.2 - 0.8 %, preferably 0.3 - 0.7 %, and most preferably 0.4 - 0.6 % of dibasic sodium phosphate. In a preferred embodiment, the phosphate salt is dibasic sodium phosphate at a concentration of 0.4 - 0.6 % (w/v). In a most preferred embodiment, the compositions contain 0.5 % (w/v) dibasic sodium phosphate.

Phosphate buffer is commonly used in aqueous pharmaceutical compositions formulated near neutral pH. Phosphate buffer (pKa₁ = 2.12, pKa₂ = 7.1, pKa₃ = 12.67) would not normally be chosen for an aqueous composition with a target pH range of 3.5 - 3.95 because it has low buffer capacity in that region. Other buffering agents are commonly used in aqueous pharmaceutical compositions, including acetate, citrate and borate buffers, but are not suitable for use in the topical nasal compositions of the present invention. Borate buffers are not suitable because they do not have any significant buffer capacity in the pH range 3.5 - 3.95. Though acetate and citrate buffers have buffer capacity in this region, they are not preferred because they have the potential to cause irritation to nasal mucosal tissues and undesirable taste and/or smell.

In addition to olopatadine and phosphate salt, the compositions of the present invention comprise sodium chloride as a tonicity-adjusting agent. The compositions contain sodium chloride in an amount sufficient to cause the final composition to have a nasally acceptable osmolality, preferably 240 - 350 mOsm/kg. Most preferably, the amount of sodium chloride in the compositions of the present invention is an amount sufficient to cause the compositions to have an osmolality of 260 - 330 mOsm/kg. In a preferred embodiment, the compositions contain 0.3 - 0.6 % sodium chloride. In a more preferred embodiment, the compositions contain 0.35 - 0.55 % sodium chloride, and in a most preferred embodiment, the compositions contain 0.35 - 0.45 % sodium chloride.

The compositions of the present invention also contain a pharmaceutically acceptable pH-adjusting agent. Such pH-adjusting agents are known and include, but are not limited to, hydrochloric acid (HCl) and sodium hydroxide (NaOH). The compositions of the present invention preferably contain an amount of pH-adjusting agent sufficient to obtain a composition pH of 3.5 - 3.95, and more preferably, a pH of 3.6 - 3.8.

In one embodiment, the aqueous compositions of the present invention consist essentially of olopatadine, phosphate buffer, sodium chloride, a pH-adjusting agent, and water, and have a pH from 3.5 - 3.95. These compositions can be manufactured as sterile compositions and packaged in multi-dose, pressurized aerosol containers to avoid microbial contamination. In another embodiment, the aqueous compositions of the present invention contain a preservative and a chelating agent such that the compositions pass United States Pharmacopeia/National Formulary XXX criteria for antimicrobial effectiveness, and more preferably the Pharm. Eur. 5th Edition criteria for antimicrobial preservation (Pharm. Eur. B preservative effectiveness standard). Suitable preservatives include p-hydroxybenzoic acid ester, benzalkonium chloride, benzododecinium bromide, and the like. Suitable chelating agents include sodium edetate and the like. The most preferred preservative ingredient for use in the compositions of the present invention is benzalkonium chloride ("BAC"). The amount of benzalkonium chloride is preferably 0.005 - 0.015 %, and more preferably 0.01 %. The most preferred chelating agent is edetate disodium ("EDTA"). The amount of edetate disodium in the compositions of the present invention is preferably 0.005 - 0.015 %, and more preferably 0.01 %.

The aqueous solution compositions of the present invention do not contain a polymeric ingredient intended to enhance the solubility of olopatadine or the physical stability of the solution. For example, the compositions of the present invention do not contain polyvinylpyrrolidone, polystyrene sulfonic acid, polyvinyl alcohol, polyvinyl acrylic acid, hydroxypropylmethyl cellulose, sodium carboxymethyl cellulose or xanthan gum.

The compositions of the present invention are preferably packaged in opaque plastic containers. A preferred container is a high-density polyethylene container equipped with a nasal spray pump. Preferably, the package is designed to provide the spray characteristics described in commonly-assigned, co-pending, U.S. Patent Application Publication No. 2006/011 0328.

The present invention also relates to a method of treating allergic rhinitis comprising topically administering to the nasal cavities a composition containing 0.6 % olopatadine, phosphate buffer, sodium chloride, a pH-adjusting agent, and water. The compositions optionally contain one or more preservative ingredients. Preferably, the compositions are administered such that 1200 mcg of olopatadine (e.g., 600/mcg per 100 microliter spray x two sprays) is delivered to each nostril twice per day.

Certain embodiments of the invention are illustrated in the following examples.

### Example 1: Topically Administrable Nasal Solution

**Table 1**

| Ingredient | Amount (%, w/v) |
|---|---|
| Olopatadine Hydrochloride | 0.665 ^{a} |
| Benzalkonium Chloride | 0.01 |
| Edetate Disodium, Dihydrate | 0.01 |
| Sodium Chloride | 0.41 |
| Dibasic Sodium Phosphate, Anhydrous | 0.5 |
| Hydrochloric Acid and/or Sodium Hydroxide | Adjust to pH 3.7 ± 0.1 |
| Purified Water | qs to 100 |

| | |
|---|---|
| ^{a} 0.665% w/v olopatadine hydrochloride (665 mcg/100 microliter spray) is equivalent to 0.6% w/v olopatadine as base (600 mcg/100 microliter spray). | |

An exemplary compounding procedure for the nasal composition shown in Table 1 is described as below.
1. Tare a suitable compounding vessel with magnetic stir bar. Add approximately 80% of the batch weight of purified water.
2. While stirring, add dibasic sodium phosphate (anhydrous), sodium chloride, edetate disodium, benzalkonium chloride and olopatadine HCl.
3. Add equivalent to approximately 0.55 g, 6N hydrochloric acid per 100 ml batch.
4. Allow adequate time between each addition for dissolution of each ingredient
5. Add purified water to approximately 90% of final batch weight.
6. Measure pH and adjust, if necessary, to 3.7 with 6N (and/or 1N) hydrochloric acid and 1N sodium hydroxide.
7. Adjust to final batch weight with purified water (QS).
8. Measure final pH.
9. Filter through 0.2 µm filtration membrane.

### Example 2: Effect of NaCl and Phosphate Buffer on Dissolution of Olopatadine Hydrochloride

The effect of NaCl on the dissolution rate of olopatadine hydrochloride in water was determined. NaCl caused a significant reduction in the rate of dissolution of olopatadine. With addition of Na₂HPO₄, however, the dissolution of olopatadine was dramatically improved. The complete dissolution of 0.6% olopatadine solution without Na₂HPO₄ would take at least several hours assuming that the entire amount of olopatadine would eventually dissolve, but with Na₂HPO₄ it takes less than one minute. The results are shown in Figure 2.

### Example 3: Effect of NaCl and Na₂HPO₄ on the dissolution olopatadine hydrochloride in a nasal vehicle.

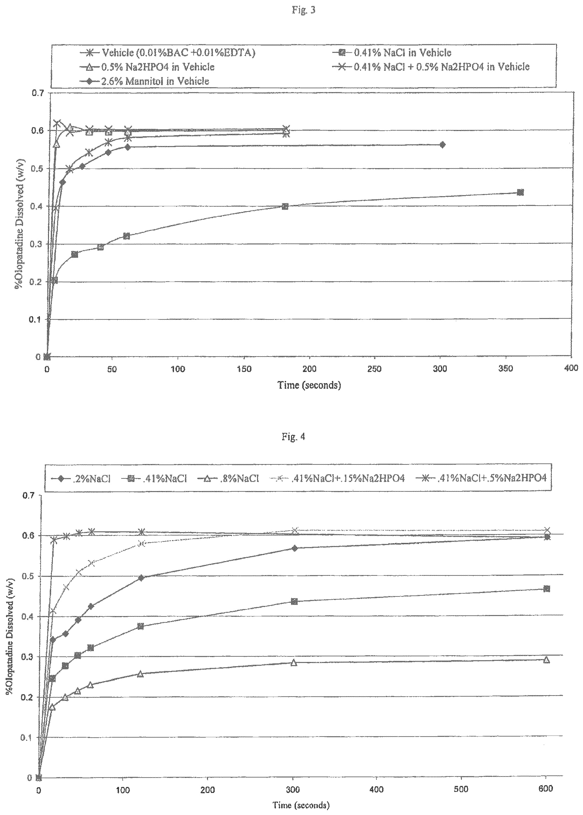

The effect of NaCl, Na2HPO4, and mannitol on the dissolution rate of olopatadine hydrochloride in a nasal formulation containing 0.01% EDTA and 0.01% BAC was determined. The results are shown in Figure 3. The effect of phosphate salt in this vehicle is the same as that shown in water in Example 2.

### Example 4: Effect of NaCl and Na₂HPO₄ Concentrations on Dissolution

The effect of NaCl and Na₂HPO₄ concentrations on the dissolution rate of olopatadine hydrochloride in a nasal formulation containing 0.01% EDTA and 0.01% BAC was determined. The results are shown in Figure 4. The aqueous solubility of olopatadine HCl decreases with increasing concentration of NaCl. However, increasing phosphate buffer correlates with increased aqueous solubility of olopatadine HCl in the presence of NaCl.

### Example 5: Effect of Phosphate Buffer on Olopatadine Nasal Spray Composition

The two compositions shown in Table 2 below were prepared using the procedure described in Example 1 and visual observations of the compositions clarity were made at different points during the compounding procedure. The results are shown in Table 2.

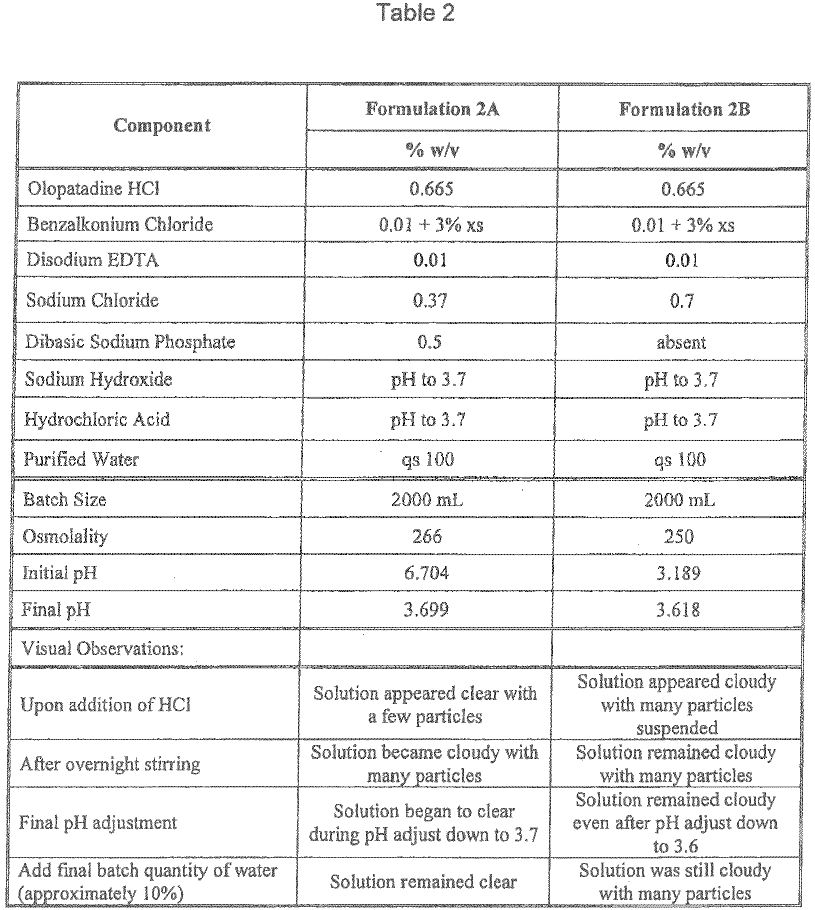

The results for Formulation A show that it is a clear solution. The results for Formulation B show that despite the pH-solubility profile indicating 0.6% olopatadine should dissolve at pH 3.189, the olopatadine did not go into solution. These results demonstrate that, without phosphate buffer, 0.665% olopatadine hydrochloride did not completely dissolve in water in the presence of 0.7% NaCl at a pH as low as 3.6 using the compounding procedure described in Example 1.

### Example 6: Effect of Phosphate Buffer Added to Cloudy 0.6 % Olopatadine Nasal Spray Composition

Formulations 3A, 3B, and 3C shown in Table 3 were prepared without phosphate buffer and, despite extensive stirring, the olopatadine HCl was not completely solubilized. A portion of Formulation 3C was removed and phosphate buffer was added to form Formulation 3D. The results, summarized in Table 3, demonstrate that 0.665% olopatadine hydrochloride is not soluble in the tested nasal vehicle without a phosphate salt.

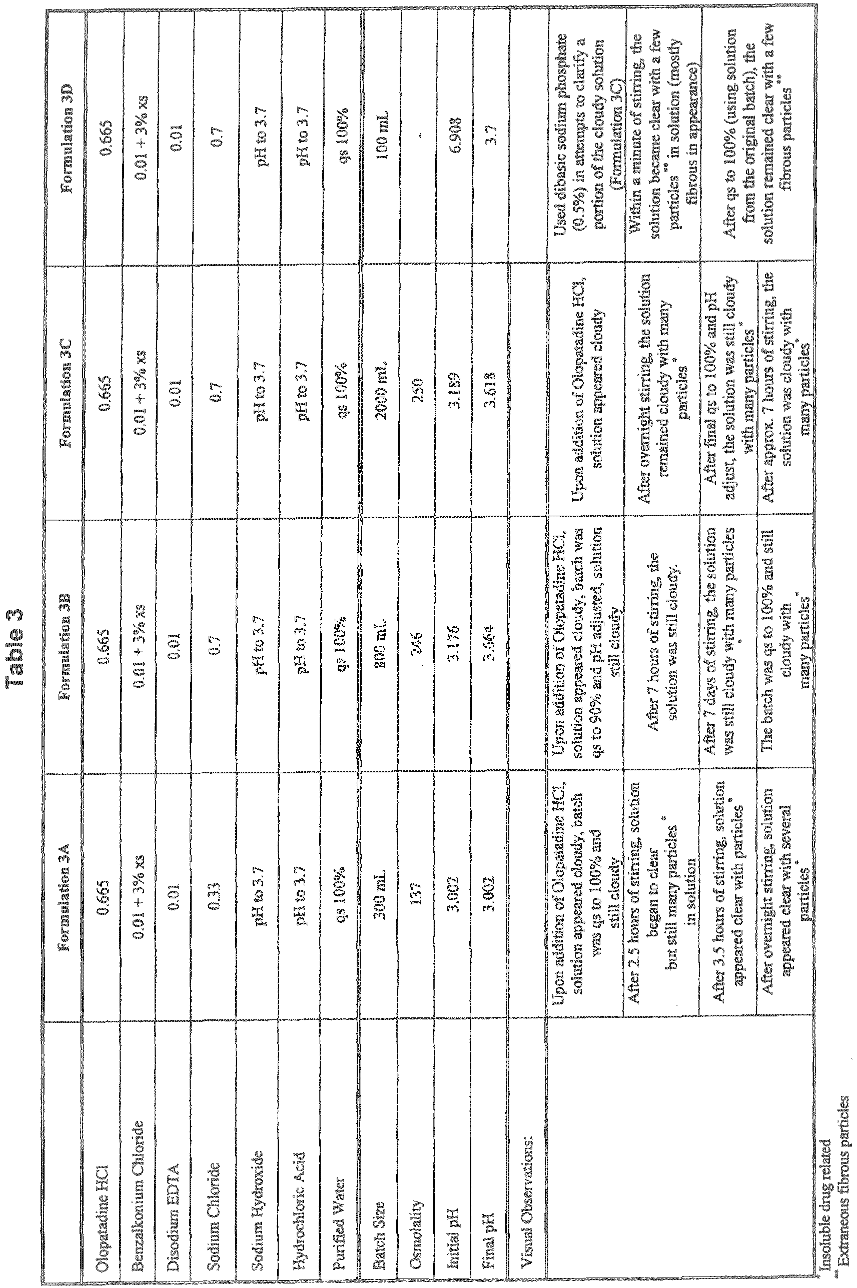

### Example 7: Effect of Compounding Sequence on 0.6 % Olopatadine Nasal Spray Composition

The composition of Example 1 above was prepared using four different sequences for the addition of ingredients. The four sequences are indicated in Table 4 in the "OA" (order of addition) columns. In each case, visual observations relating to the composition's clarity were recorded. The results are shown in Table 4. In all four cases (Formulations 4A - 4D), at the end of the compounding procedure, the solutions were clear. (The solutions contained some extraneous fibrous particles that did not appear to be related to the drug or the formulation excipients and were likely attributable to laboratory equipment and glassware.)

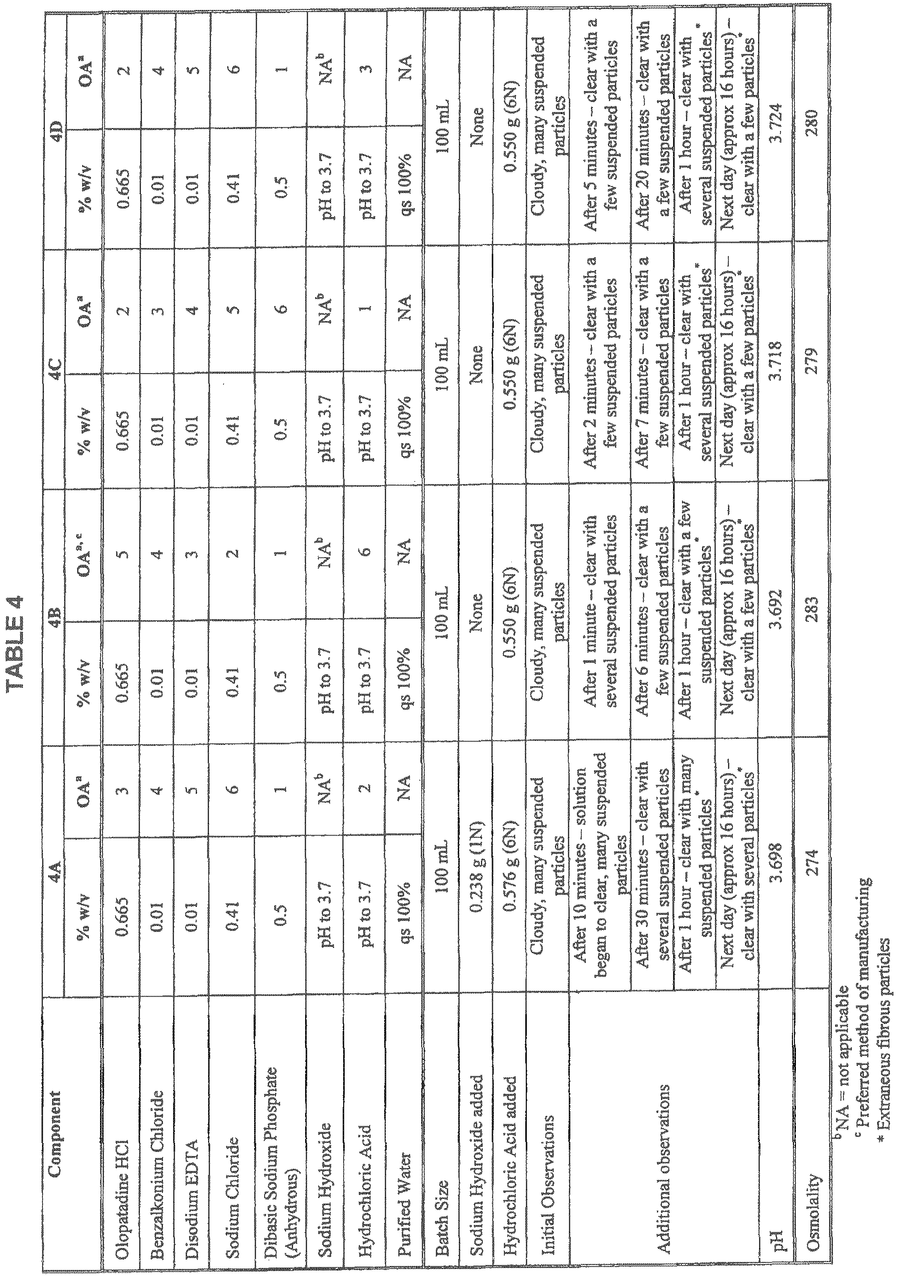

### Example 8: Effect of Various Buffer Systems

The composition of Example 1 above was prepared but acetate, borate and citrate buffers, respectively, were substituted in place of the phosphate buffer. Visual observations regarding the clarity of each of the compositions were recorded and are shown in Table 5.

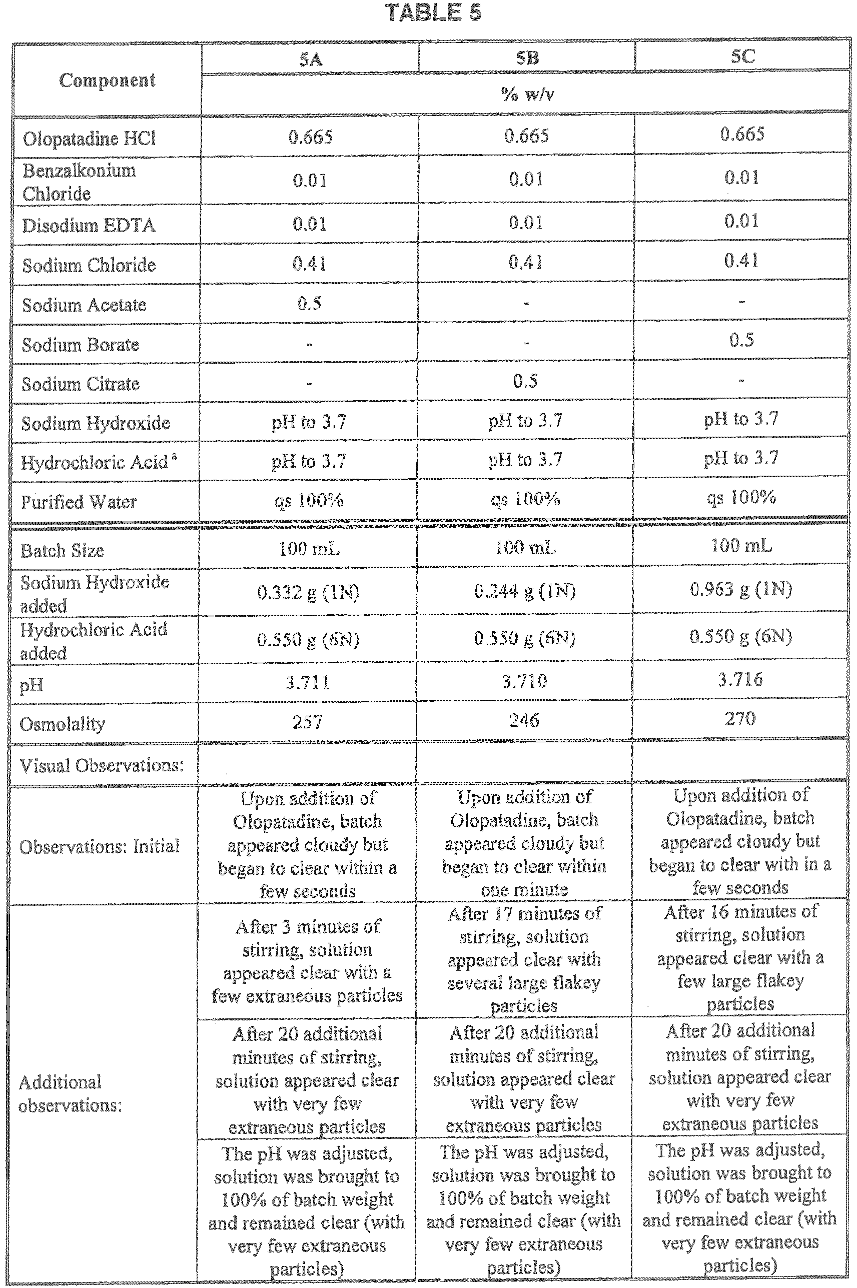

### Example 9: Effect of Phosphate Buffer, NaCl, and Hot Water

The compositions shown in Table 6 were prepared to examine (1) the effect of adding phosphate buffer to a composition containing olopatadine hydrochloride, BAC, EDTA, NaOH/HCl, and NaCl, (2) the effect of adding NaCl to a composition containing olopatadine, BAC, EDTA, NaOH/HCl, and (3) the effect of hot water on the dissolution of olopatadine in a composition comprising olopatadine, BAC, EDTA, NaCl and NaOH/HCI. In each case, visual observations concerning the clarity of the composition were recorded. The results are shown in Table 6.

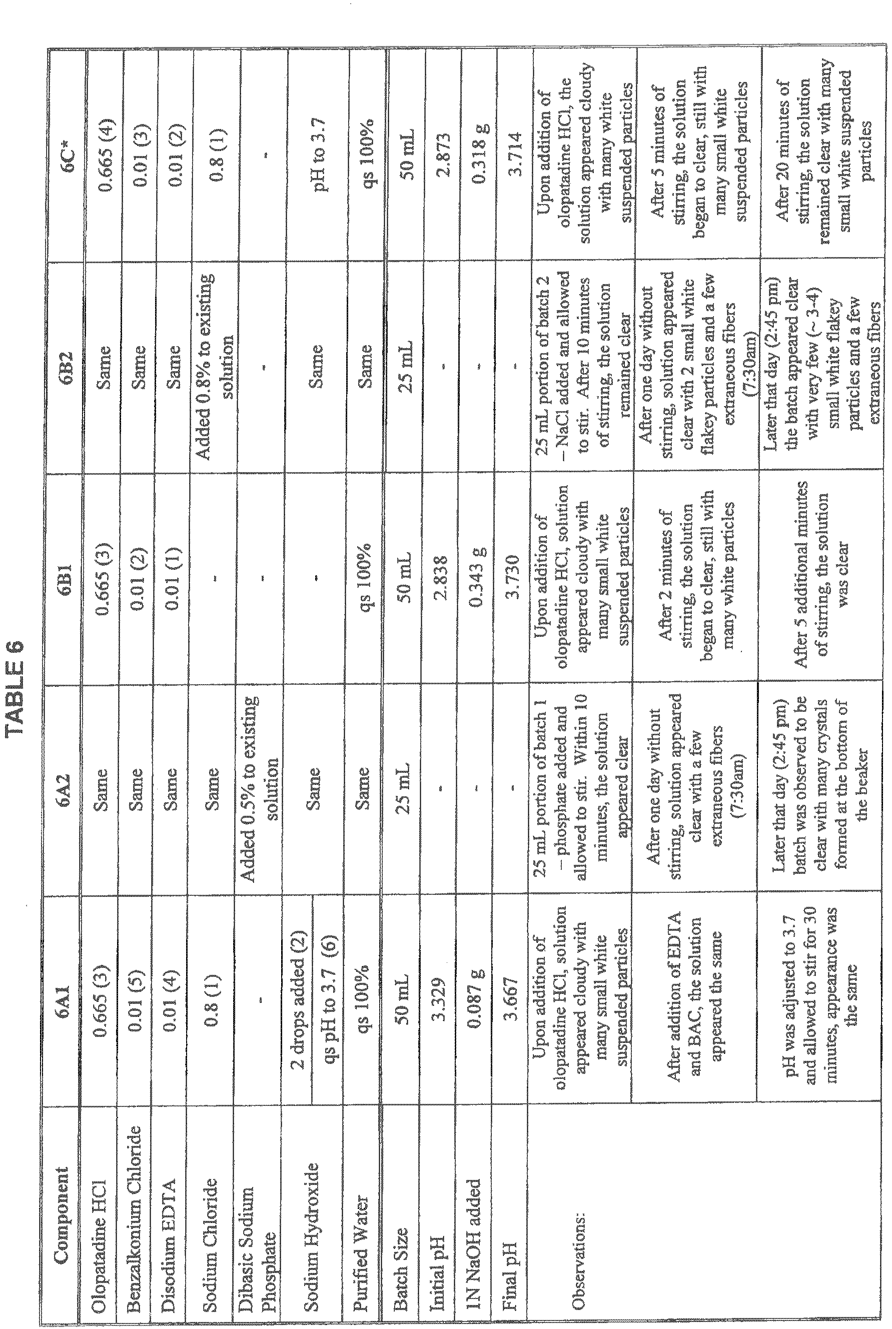

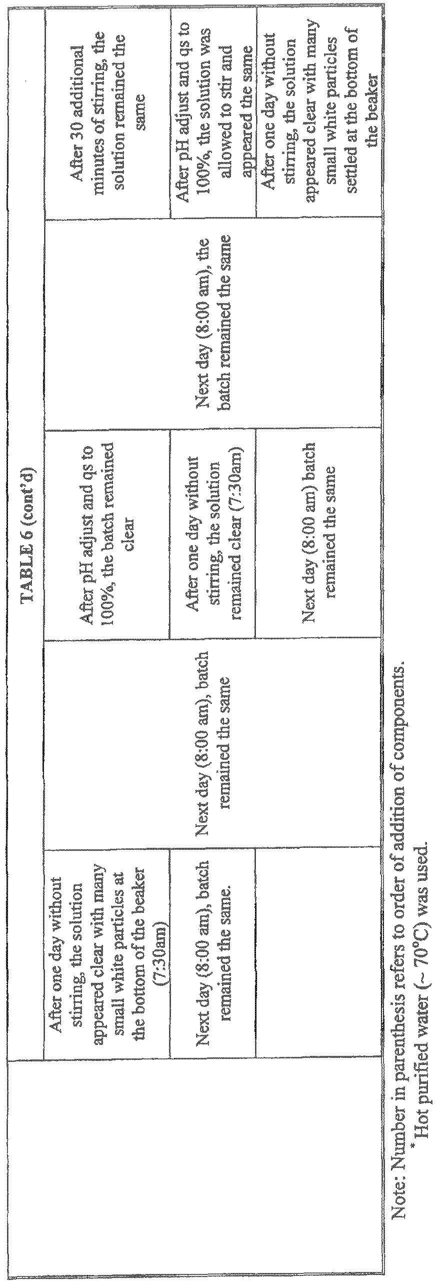

### Example 10: Buffer Capacity of Phosphate Buffer

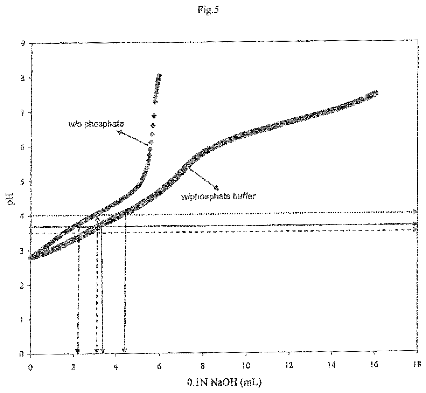

The contribution of phosphate buffer to the buffer capacity of the composition of Example 1 was determined in a classical acid-base titration experiment. The results are shown in Figure 5. The buffer capacity of the composition of Example 1 (without phosphate buffer) was 2.66 from pH 3.5 - 3.8 and 2.7 from pH 3.5 - 3.9. The buffer capacity of the composition of Example 1 (i.e., including phosphate buffer) was 2.93 from pH 3.5 - 3.8 and 3.1 from pH 3.5 - 3.8.

### Example 11: Stability of Olopatadine Nasal Spray Compositions Lacking Phosphate Buffer

The compositions (without phosphate buffer) shown below in Table 7A were prepared. Visual observations of the clarity of each composition were recorded as each composition was prepared. The results are shown in Table 7A.

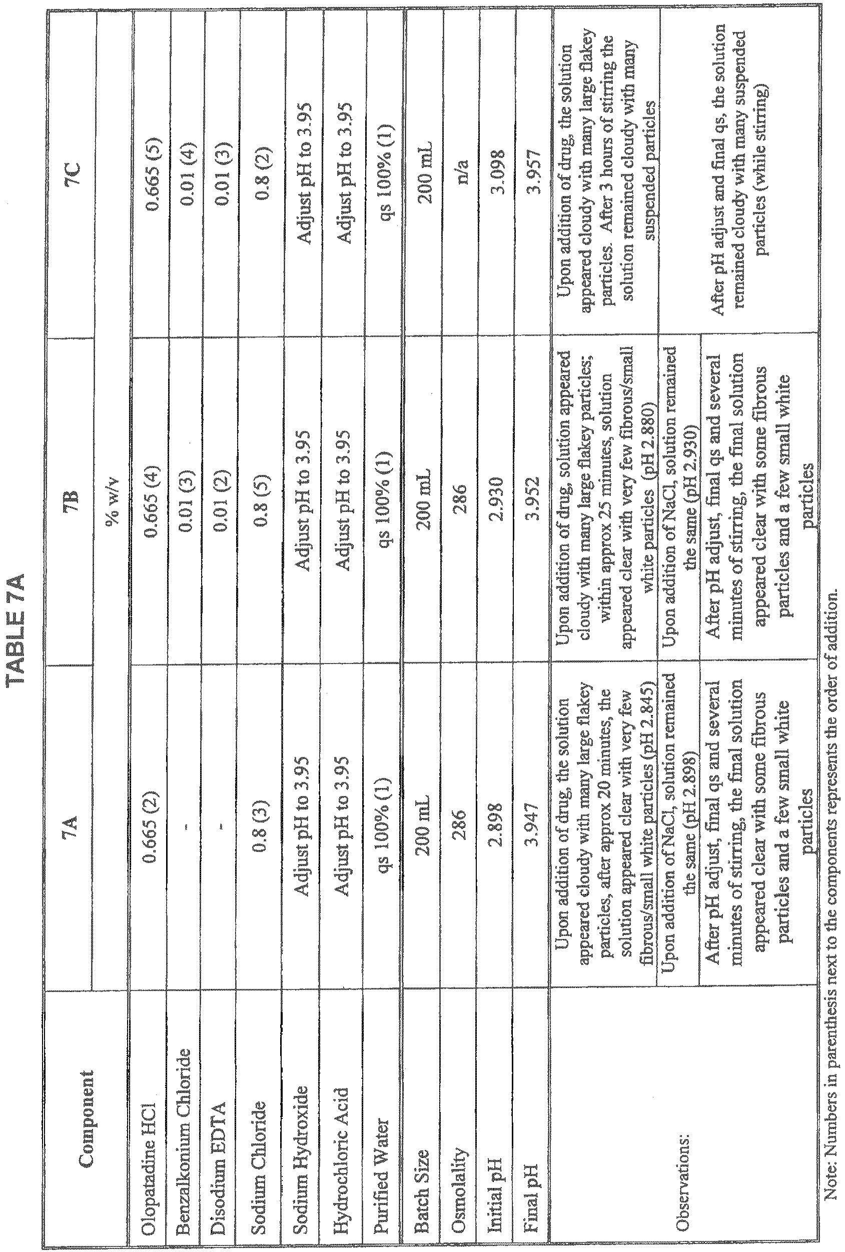

Each of the compositions was then split. One portion of each was split again into three storage batches ("pre-filtration") and the other portion was filtered through a 0.2 µM filter and then split into three storage batches ("post-filtration"). One of the storage batches of each set was stored at room temperature (-22 °C), one in the refrigerator (∼4 °C), and one subjected to freeze-thaw cycling (one day in the freezer (∼ -20 °C) and one day at room temperature, except over the weekends). Visual observations of the clarity of each sample of Formulation 7A (lacking BAC and EDTA) were recorded on the indicated days and the results were recorded. The results are shown in Tables 7B (pre-filtration) and 7C (post-filtration).

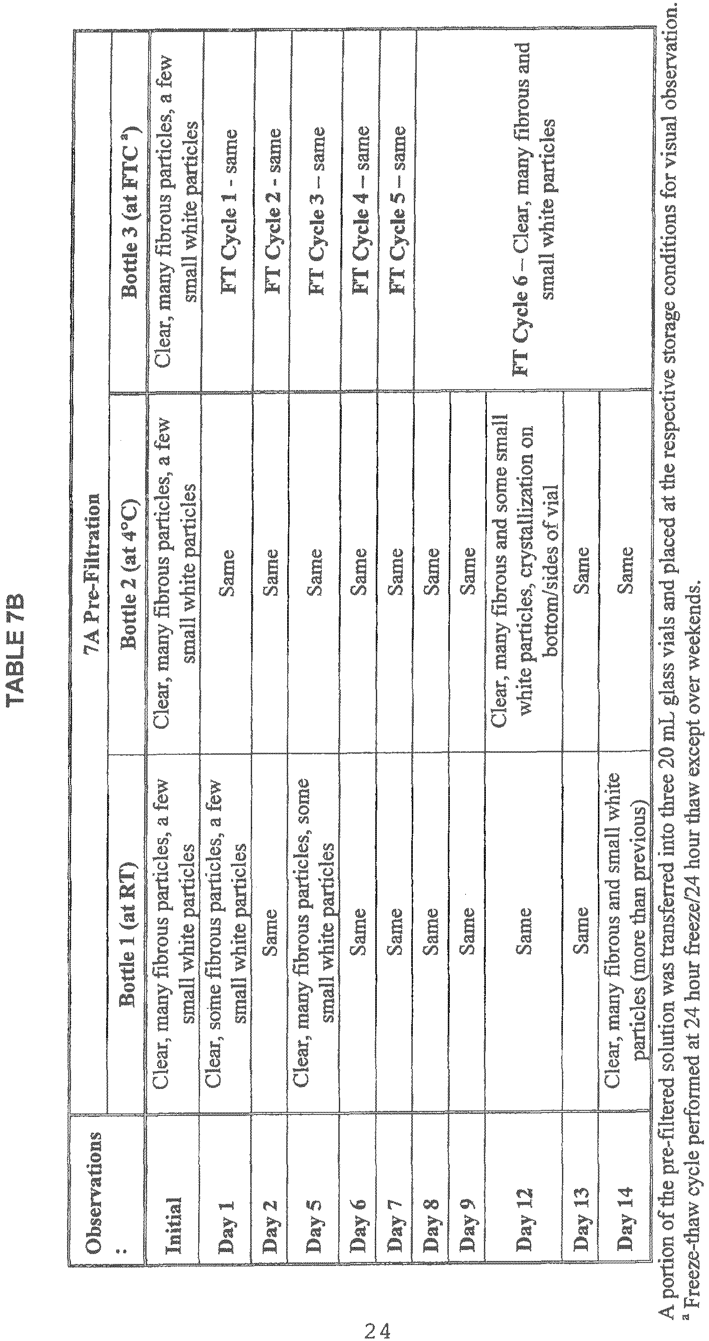

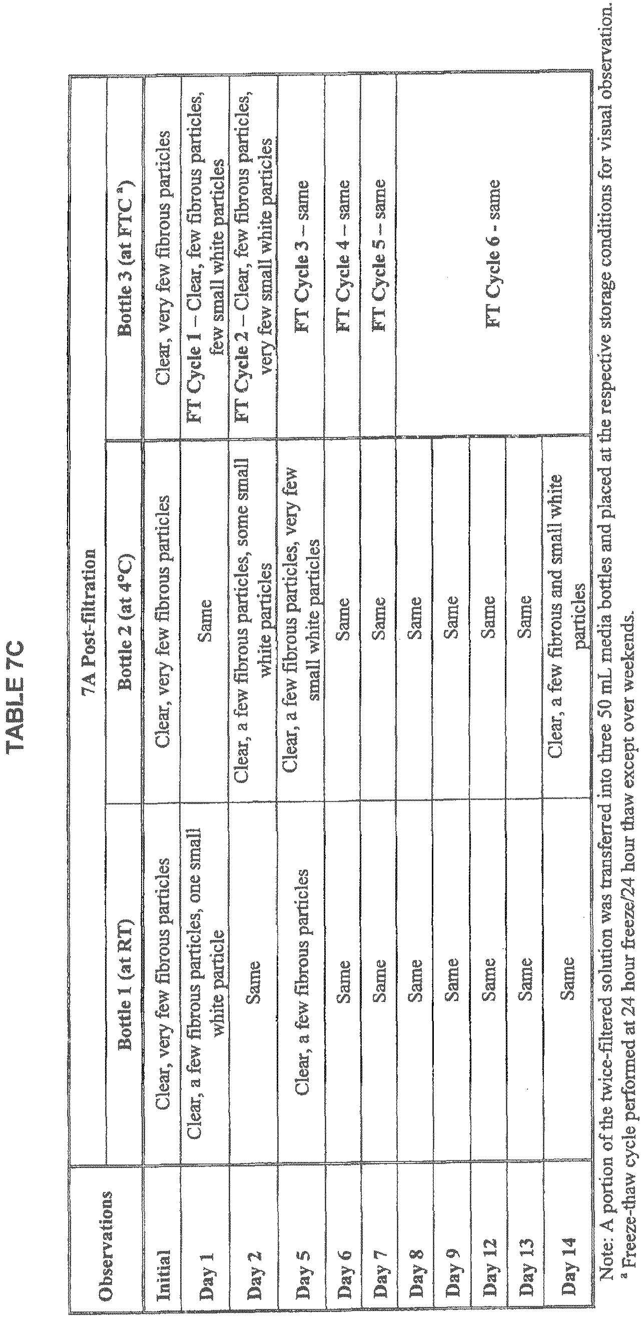

After nine days of observation, the post-filtration portion of Formulation 7A was split and a stir bar was added to each sample as a seeding agent (the stir bar was not rotating). Visual observations were recorded and the results are shown in Table 7 D.

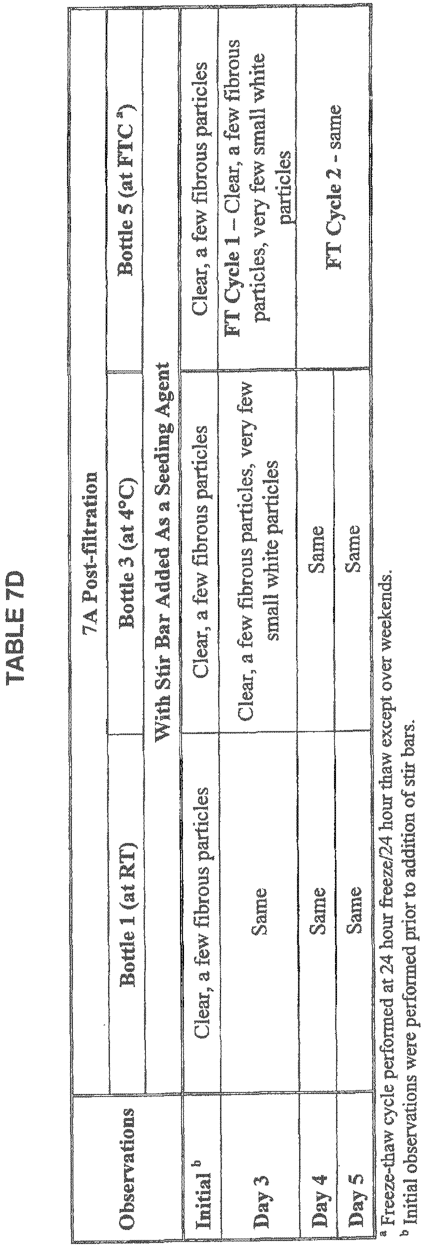

To other portions of composition 7A split after nine days of observation, excess olopatadine (a few small granules) was added to both the pre-filtration and post-filtration samples to determine if seeding would cause olopatadine to precipitate. Visual observations were recorded on the indicated days. The results are shown in Tables 7 E (unfiltered composition) and 7 F (filtered composition).

**TABLE 7E**

| **Observations** | **7A Pre-filtration (with excess olopatadine HCl)** | |
|---|---|---|
| | **Bottle 1 (at RT)** | **Bottle 2 (at FTC ^{a})** |
| **Initial ^{b}** | Clear, many fibrous particles, some small white particles | Clear, many fibrous particles, some small white particles |
| **Day 3** | Clear, many fibrous/small white particles (powdery settling) | **FT Cycle 1 -** clear, many fibrous/small white particles (powdery settling) |
| **Day 4** | Same | **FT Cycle 2** - same |
| **Day 5** | Same | |

| | | |
|---|---|---|
| ^{a} Freeze-thaw cycle performed at 24 hour freeze/24 hour thaw except over weekends. ^{b} Initial observations were performed prior to addition of excess olopatadine HCl. | | |

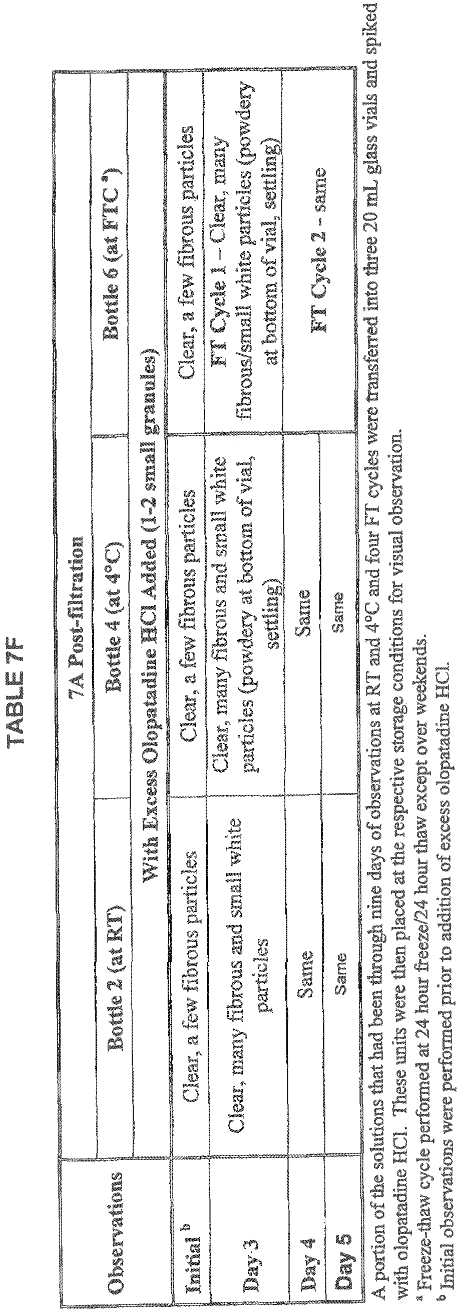

The stability of the composition "7B" (containing BAC and EDTA) was evaluated in the same fashion. The results are shown in Tables 7G (Pre-filtration), 7H (Post-filtration), 7I (with stir bar added after 9 days), 7J (with excess olopatadine added after 9 days; pre-filtration), and 7K (with excess olopatadine added after 9 days; post-filtration).

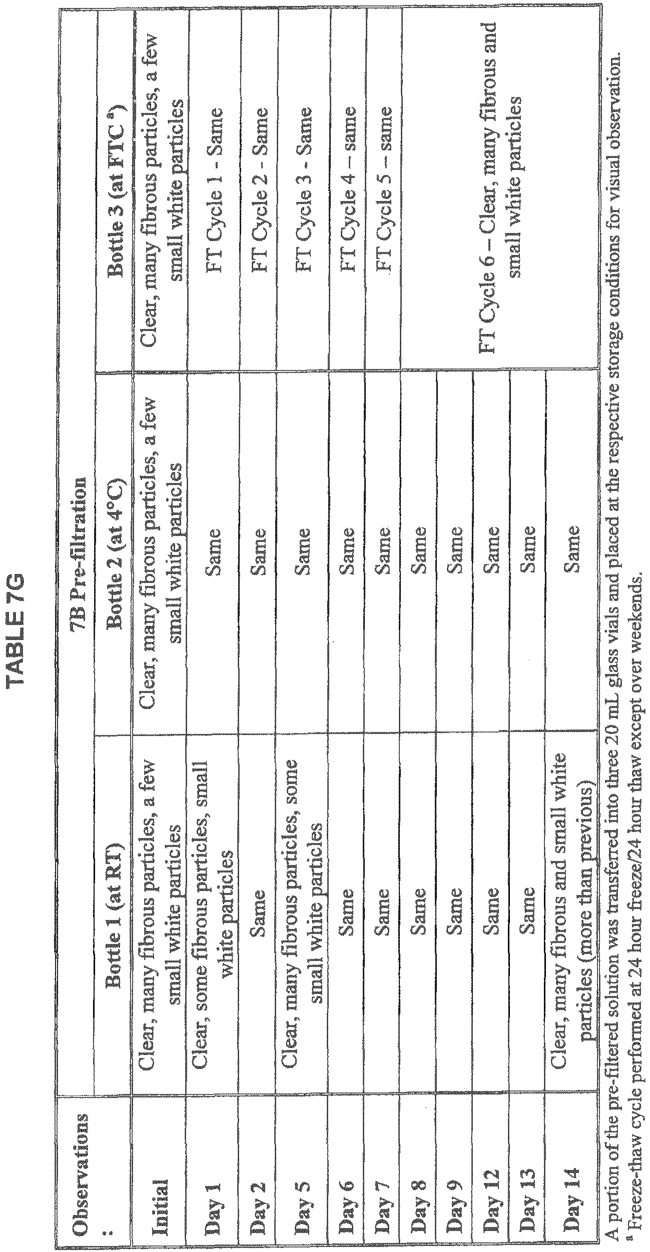

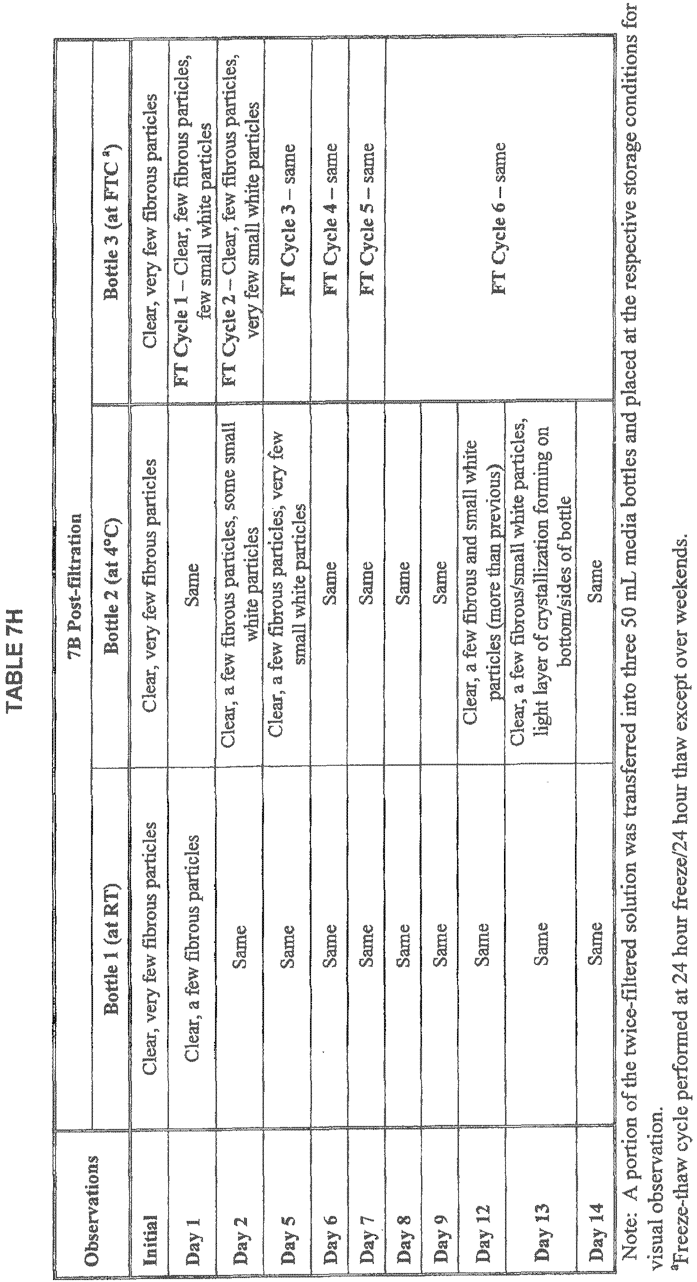

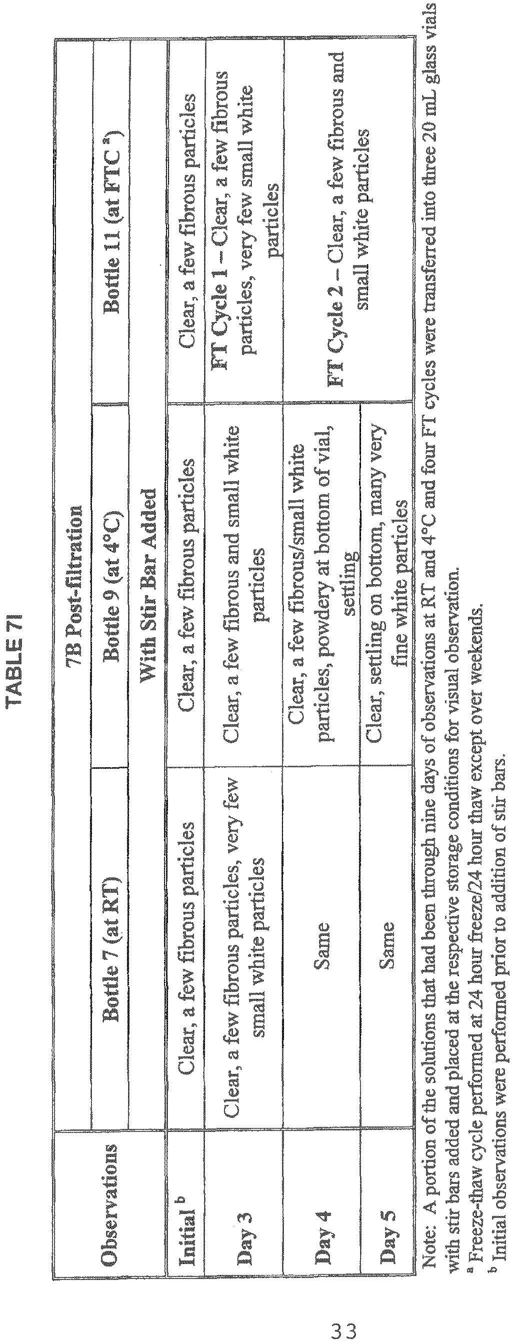

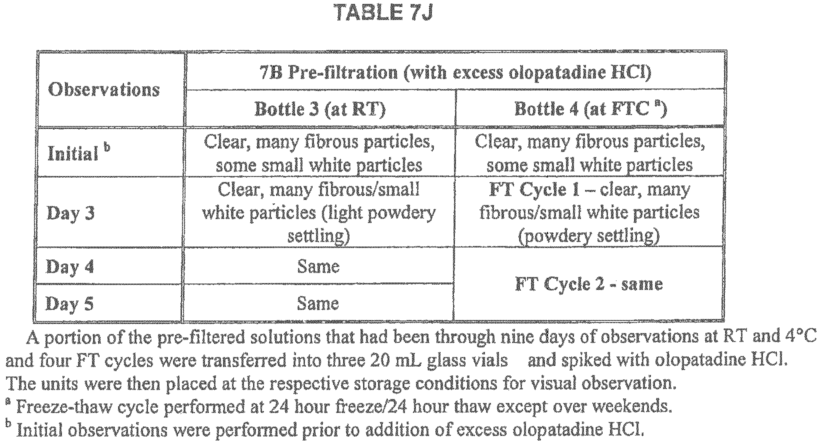

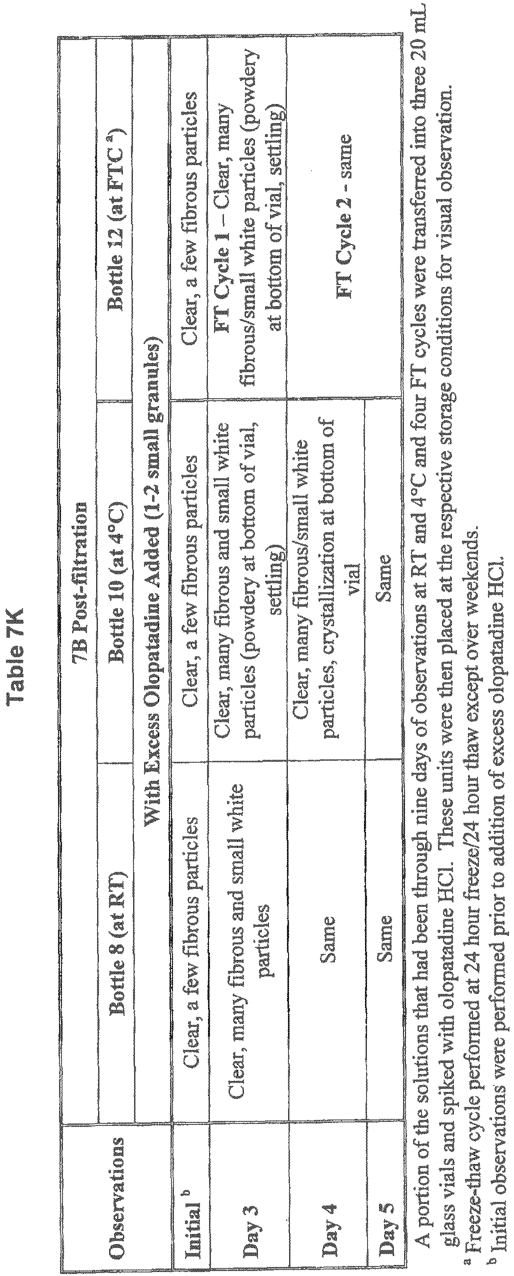

### Example 12: Effect of Phosphate Buffer

The compositions shown below in Table 8 were prepared using a compounding procedure similar to that described in Example 1. In all four cases, the NaCl was added after olopatadine during the compounding. All four compositions contained the equivalent of 110% of a 0.6% targeted concentration. Two of the compositions were formulated at a pH of 3.95 and two at 4.10 to test an extreme condition. The results are shown in Table 8.

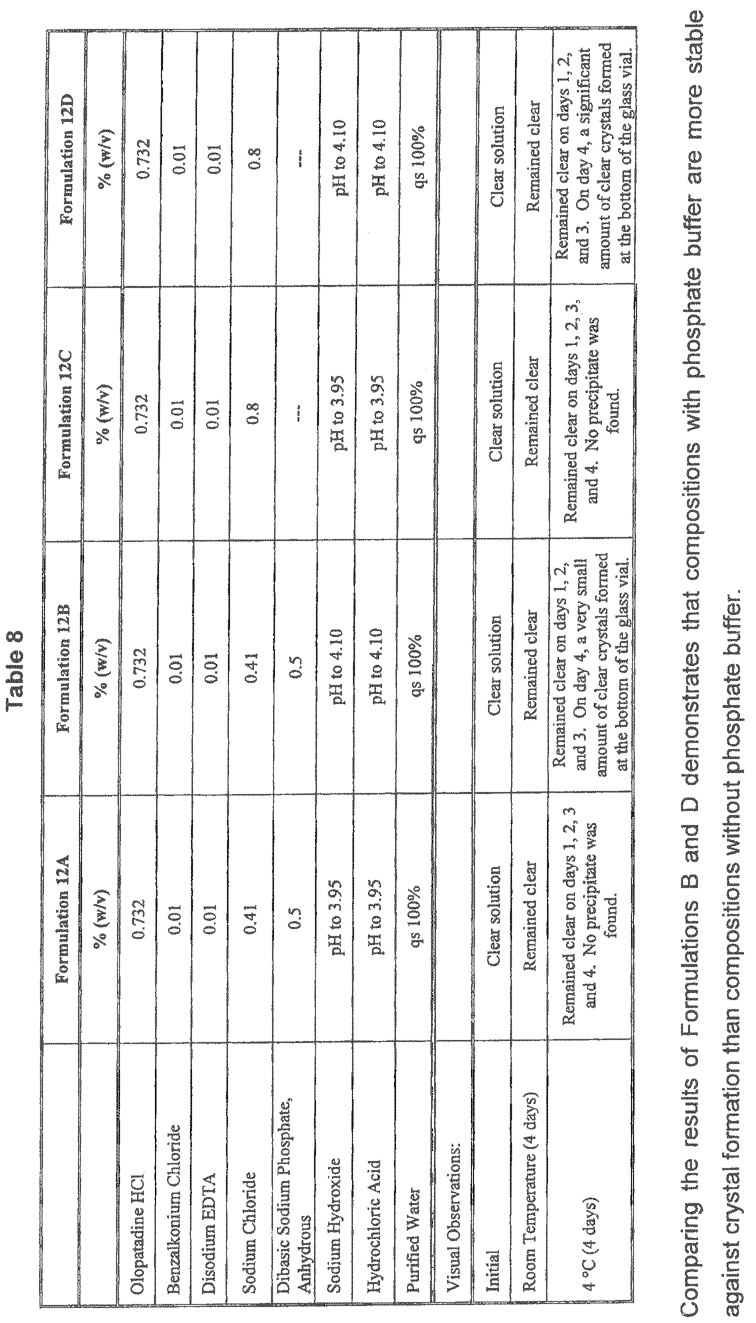

### Example 13: Storage Stability

The solution stability of the composition of Example 1 was examined by preparing variations of the composition at the pH's shown in Table 9 and subjecting the samples to 13 freeze-thaw cycles (same cycles as described in Example 11 above). Following the last cycle, the samples were stored in the freezer for approximately three weeks and then analyzed. The amount of olopatadine (pre- and post-filtration, 0.2 µM filter) was determined by HPLC assay as a percent of the labeled amount (0.6%). The samples were evaluated using four tests of solution clarity: "Nephelos" values were obtained using a turbidimeter (HF Scientific, Inc., Model No. DRT100B); "Clarity" was determined by visual observation using a method similar to the Ph. Eur. (5th Edition) method for evaluating solution clarity and degree of opalescence; "Precipitate" was determined by visual inspection and the presence of absence of precipitates was recorded; "Particles by Visual Observation" was determined by visual inspection under a light box where not more than 3 particles per 5 mL sample is considered "essentially particle free." Osmolality and pH were also determined for each composition. The results are shown in Table 9. In four of the five cases (Samples 1 - 4), the compositions were clear solutions following the freeze-thaw cycling study, demonstrating the composition of Example 1 is a stable aqueous solution despite the absence of a polymeric physical stability-enhancing agent. The sample that did not remain a clear solution is Sample 5 (pH = 4.45).

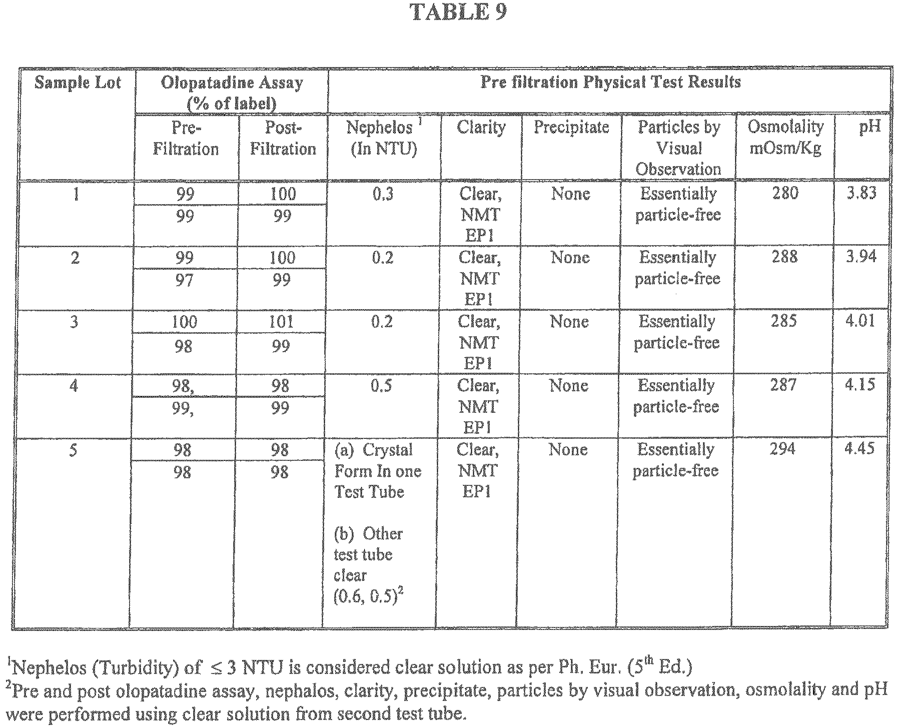

This invention has been described by reference to certain preferred embodiments; however, it should be understood that it may be embodied in other specific forms or variations thereof without departing from its special or essential characteristics. The embodiments described above are therefore considered to be illustrative in all respects and not restrictive, the scope of the invention being indicated by the appended claims rather than by the foregoing description.

## Claims

1. A composition consisting essentially of
a) 0.54 - 0.62 % (w/v) olopatadine free base or an equivalent amount of a pharmaceutically acceptable salt of olopatadine;
b) a phosphate salt in an amount equivalent to 0.2 - 0.8 % (w/v) dibasic sodium phosphate, wherein the phosphate salt selected from the group consisting of monobasic sodium phosphate; dibasic sodium phosphate; tribasic sodium phosphate; monobasic potassium phosphate; dibasic potassium phosphate; and tribasic potassium phosphate;
c) 0.3 - 0.6 % (w/v) NaCl;
d) a pH-adjusting agent in an amount sufficient to cause the composition to have a pH of 3.5 - 3.95;
e) 0.005 - 0.015 % (w/v) benzalkonium chloride;
f) 0.005 - 0.015 % (w/v) edetate disodium; and
g) water.

2. The composition according to claim 1 consisting essentially of
a) 0.6 % (w/v) olopatadine free base or an equivalent amount of a pharmaceutically acceptable salt of olopatadine;
b) 0.4 - 0.6 % (w/v) dibasic sodium phosphate;
c) 0.35 - 0.45 % (w/v) NaCl;
d) a pH-adjusting agent in an amount sufficient to cause the composition to have a pH of 3.5 - 3.95, wherein the pH-adjusting agent is selected from the group consisting of NaOH and HCl;
e) 0.01 % (w/v) benzalkonium chloride;
f) 0.01 % (w/v) edetate disodium; and
g) water.

## Patentansprüche

1. Zusammensetzung, im Wesentlichen bestehend aus
a) 0,54 - 0,62% (w/v) Olopatadin freie Base oder eine äquivalente Menge eines pharmazeutisch unbedenklichen Salzes von Olopatadin,
b) ein Phosphatsalz in einer 0,2 - 0,8% (w/v) Dinatriumhydrogenphosphat äquivalenten Menge, wobei das Phosphatsalz aus der aus Natriumdihydrogenphosphat, Dinatriumhydrogenphosphat, Natriumphosphat, Kaliumdihydrogenphosphat, Dikaliumhydrogenphosphat und Kaliumphosphat bestehenden Gruppe ausgewählt ist,
c) 0,3 - 0,6% (w/v) NaCl,
d) einem Mittel zum Einstellen des pH-Wertes in einer Menge, die ausreicht, um den pH-Wert der Zusammensetzung auf 3,5 - 3,95 einzustellen,
e) 0,005 - 0,015% (w/v) Benzalkoniumchlorid,
f) 0,005 - 0,015% (w/v) Dinatriumedetat und
g) Wasser.

2. Zusammensetzung nach Anspruch 1, im Wesentlichen bestehend aus
a) 0,6% (w/v) Olopatadin freie Base oder eine äquivalente Menge eines pharmazeutisch unbedenklichen Salzes von Olopatadin,
b) 0,4 - 0,6% (w/v) Dinatriumhydrogenphosphat,
c) 0,35 - 0,45% (w/v) NaCl,
d) einem Mittel zum Einstellen des pH-Wertes in einer Menge, die ausreicht, um den pH-Wert der Zusammensetzung auf 3,5 - 3,95 einzustellen, wobei das Mittel zum Einstellen des pH-Wertes aus der aus NaOH und HCl bestehenden Gruppe ausgewählt ist,
e) 0,01% (w/v) Benzalkoniumchlorid,
f) 0,01% (w/v) Dinatriumedetat und
g) Wasser.

## Revendications

1. Composition essentiellement constituée de
a) 0,54 à 0,62 % (poids/volume) d'olopatadine sous forme de base libre ou d'une quantité équivalente d'un sel pharmaceutiquement acceptable d'olopatadine ;
b) un sel de phosphate en une quantité équivalente à 0,2 à 0,8 % (poids/volume) de phosphate de sodium dibasique, le sel de phosphate étant choisi dans le groupe constitué par le phosphate de sodium monobasique ; le phosphate de sodium dibasique ; le phosphate de sodium tribasique ; le phosphate de potassium monobasique ; le phosphate de potassium dibasique ; et le phosphate de potassium tribasique ;
c) 0,3 à 0,6 % (poids/volume) de NaCl ;
d) un agent d'ajustement du pH en une quantité suffisante pour amener la composition à posséder un pH de 3,5 à 3,95 ;
e) 0,005 à 0,015 % (poids/volume) de chlorure de benzalkonium ;
f) 0,005 à 0,015 % (poids/volume) d'édétate disodique ; et
g) d'eau.

2. Composition selon la revendication 1 essentiellement constituée de
a) 0,6 % (poids/volume) d'olopatadine sous forme de base libre ou d'une quantité équivalente d'un sel pharmaceutiquement acceptable d'olopatadine ;
b) 0,4 à 0,6 % (poids/volume) de phosphate de sodium dibasique ;
c) 0,35 à 0,45 % (poids/volume) de NaCl ;
d) un agent d'ajustement du pH en une quantité suffisante pour amener la composition à posséder un pH de 3,5 à 3,95, l'agent d'ajustement du pH étant choisi dans le groupe constitué par NaOH et HCl ;
e) 0,01 % (poids/volume) de chlorure de benzalkonium ;
f) 0,01 % (poids/volume) d'édétate disodique ; et
g) d'eau.
